# EUROPEAN PATENT SPECIFICATION

(11) **EP 0 576 835 B1**
(45) Date of publication and mention of the grant of the patent: **05.03.1997**
(21) Application number: 93108576.5
(22) Date of filing: 27.05.1993
(51) Int. Cl.: A61B 17/12

(54) **Two-part surgical ligation clip and modular ligation clip applicator**
Chirurgische zweiteilige Abbindeklammer und modularer Applikator für Abbindeklammern
Pince chirurgicale de ligature en deux pièces et applicateur modulaire de pince de ligature

(30) Priority: 30.06.1992 US 906938; 13.10.1992 US 960080
(43) Date of publication of application: 05.01.1994
(60) Divisional application: 96103415.4
(73) Proprietor: AMERICAN CYANAMID COMPANY, Stamford Connecticut 06904-0060 (US)
(72) Inventor: Conners, John A., Fairfield, Connecticut 06430 (US); Allen, William J., Stratford, Connecticut 06496 (US); Jessup, George, Strathfield, NSW 2135 (AU); Ahari, Frederick F., Southport, Connecticut 06490 (US); Crainich, Lawrence, Charlestown, New Hampshire 03603 (US)
(74) Representative: Wächtershäuser, Günter, Prof. Dr.

(56) References cited:
- EP-A- 0 178 469

## Description

The present invention relates to a surgical ligation clip typically used during a surgical procedure. More particularly, the ligation clip of the present invention provides a two-part assembly comprised of a track for gripping, for example, a blood vessel and a clip for closing the track to ligate the blood vessel.

Such a ligation clip is known from EP-A-0 178 469 on which the preamble of Claim 1 is based. However, this clip does not provide a locking means for securing the track within the clip when in closed position.

Moreover the surgical ligation clip is provided in combination with a ligation clip applicator.

More particularly, a modular applicator for positioning, securing, and closing the surgical ligation clip around a vessel to be occluded.

Ligation clips used in surgical procedures are well known in the art. For example, clips are used in surgical procedures to ligate blood vessels. Typically, a surgical ligation clip uses a clamp to compress a severed blood vessel to stop the flow of blood.

One example of a surgical ligation clip is formed of two substantially symmetrically-shaped arms connected together at an apex and a U-shaped member for manipulating the arms. Each arm comprises a more flexible portion and a bend at the more flexible portion. The surgical clip is positioned around the blood vessel to be ligated and the arms are manipulated to form a diamond-shaped enclosure around the blood vessel. The apex of the arms is slid within the U-shaped member to collapse the arms at the more flexible portions and tightly compress the blood vessel therebetween. The surgical ligation clip is made from a non-metallic material such as a synthetic bioabsorbable polymer with, for example, a glycolic and ester linkage.

While the noted clip incorporates many advantageous features, still further improvements in surgical ligation clips are desirable.

There are also many types of known mechanical applicators for closing a conventional ligation clip around a vessel. Several examples of known applicators include the so-called push-pull type, which uses a plunger or a piston, or a fulcrum type applicator having a scissors-like or pliers-like handle.

Other examples of devices for applying metal ligation clips include movable jaws which close together to crimp the clip about a vessel.

However, further improvements for applying a surgical ligation clip are desirable, and in particular, a ligation clip applicator is beneficial for use with unique two-part ligation clips. As noted, those ligation clips provide many advantages over known metal clips since they provide superior clamping strength and may be made of bioabsorbable materials.

It is an object of the present invention to provide an improved surgical ligation clip.

It is another object of the present invention to provide a surgical ligation clip with a track for better gripping the blood vessel to be ligated.

It is a further object of the present invention to provide a track and a clip which cooperate with each other to lock the track in an open position.

It is still a further object of the present invention to provide a track and a clip which cooperate with each other to lock the track in a closed position.

It is yet another object of the invention to provide a clip with increased strength for tightly securing the blood vessel within the closed track.

In accordance with the invention as defined in claim 1, a surgical ligation clip comprises a track having two arms for engaging around a vessel to be ligated, with the arms being joined together at a proximal end of the track. A clip has an extended slot for slidably receiving the track. In addition, first locking means locks the track in a closed position in the clip, and second locking means locks the track in an open position in the clip.

It is also an object of the present invention as defined in claim 1 to provide an improved ligation clip applicator.

It is another object of the present invention to provide a ligation clip applicator for use with a two-part ligation clip.

It is another object of the present invention to provide a ligation clip applicator that can apply a ligation clip using minimally invasive surgery.

It is still another object of the present invention to provide a ligation clip applicator that can be used proficiently to manipulate a mounted ligation clip and place it in position about the vessel to be ligated.

Preferably the surgical ligation clip comprises in combination a ligation clip applicator which comprises a module attached to the distal end of a hollow shaft of the applicator for supporting a two part ligation clip, the module including holding means for holding a first part of the ligation clip in a stationary position relative to said applicator and sliding means for engaging and advancing a second part of the ligation clip relative to said first part to close the first part, and actuation means, mountable with said module, for actuating said sliding means.

The ligation clip applicator comprises an elongated hollow shaft housing a sliding advancer, and a handle assembly connected to a proximal end of the shaft and having actuating means for actuating the sliding advancer. A module is connected to a distal end of the shaft and includes holding means for holding a first part of the ligation clip in a stationary position and sliding means for advancing another part of the ligation clip forwardly about the first part.

Furthermore the ligation clip applicator comprises a handle assembly having a squeezable trigger and an elongated shaft connected at its proximal end to the handle assembly. The elongated shaft houses a sliding advancer that is actuated by squeezing the trigger to move in the longitudinal direction of the shaft. In addition, a module is connected to a distal end of the elongated shaft for supporting a ligation clip. The module has a cartridge housing, pivotable latches mounted within the housing and a sliding pusher slidable within the housing by actuation of the advancer.

These and other objects, aspects, features and advantages of the present invention will become apparent from the following detailed description of the preferred embodiments taken in conjunction with the accompanying drawings.
Figures 1 to 3 are perspective views of the surgical ligation clip showing how it is positioned around the blood vessel to be ligated during different stages of use in accordance with the present invention;
Figure 4 is a top plan view of the track in accordance with the present invention;
Figure 5 is a side elevational view of the track in accordance with the present invention;
Figure 6 is an end elevational view of the track in accordance with the present invention;
Figure 7 is a partial top plan view, partially in cross-section, of the track in accordance with the present invention; and
Figure 8 is a top plan view of the clip in accordance with the present invention.
Figure 9 is a side elevational view of the ligation clip applicator in accordance with the present invention;
Figure 10 is an exploded perspective view of a handle assembly arid a proximal end of a neck portion of the ligation clip applicator;
Figure 11 is a perspective view of an advancing mechanism of the ligation clip applicator;
Figure 12 is a side elevational view, partly in vertical cross-section, of the ligation clip applicator showing a ligation clip module and the distal end of the neck portion;
Figure 13 is a vertical cross-sectional view of the proximal end of the ligation clip applicator showing the handle assembly in three different positions;
Figure 14 is a perspective view of the distal end of the ligation clip applicator;
Figure 15 is an exploded perspective view of the ligation clip module of the ligation clip applicator;
Figure 16 is a perspective view of the distal end of the ligation clip applicator showing the ligation clip loaded in the ligation clip module and ready to be fired;
Figure 17 is a perspective view of the distal end of the ligation clip applicator showing the advancing mechanism in the actuated position and the ligation clip closed around the vessel; and
Figure 18 is a perspective view of the distal end of the ligation clip applicator showing the advancing mechanism in the retracted position and the ligation clip closed about a vessel and released from the ligation clip module.

For convenience of reference, as used herein, the term "distal" will refer to that part of the device which is farthest away from the surgeon-user, and the term "proximal" refers to that part of the device which is closest to the surgeon-user.

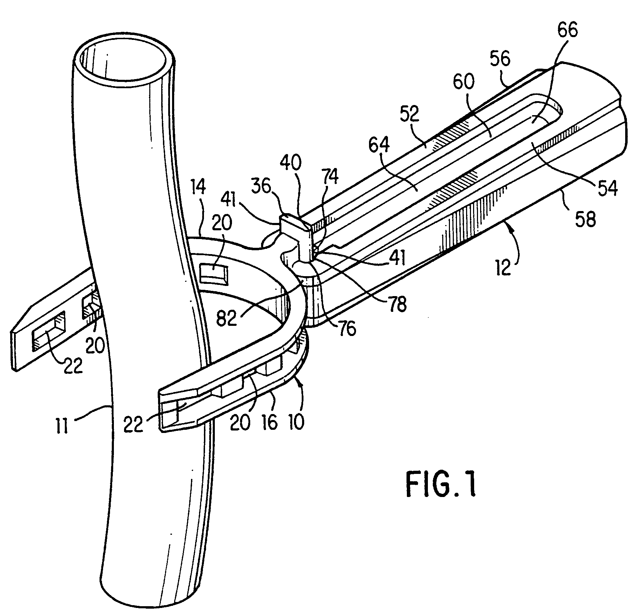

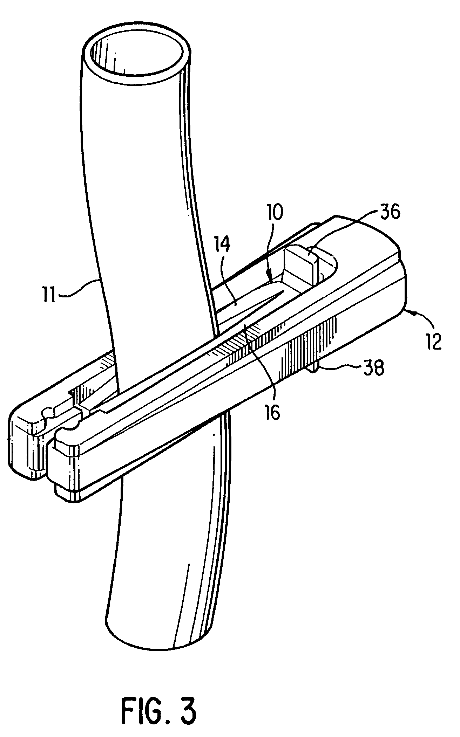

Figure 1 shows a preferred embodiment of the surgical ligation clip of the subject invention. The two main components of the ligation clip are a track 10 and a clip 12, which are preferably made of a non-metallic resilient polymer-type material. The track is relatively flexible while the clip is made of a relatively rigid material. Generally speaking, the track is connected to the clip and positioned around, for example, a blood vessel 11 to be ligated. The track is held stationary and the clip is forced forwardly so the track and clip slide relative to each other. This sliding motion closes and locks the track about the blood vessel as shown in Figure 3.

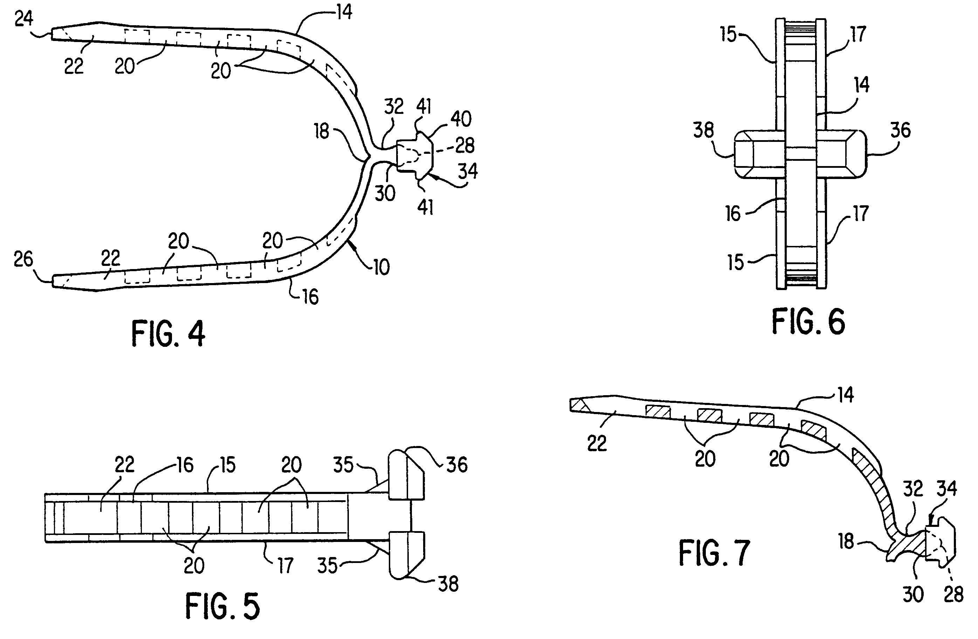

The track 10 will be discussed in detail with reference to Figures 4 through 7. The track is comprised of two curved arms 14 and 16 positioned to form a C-shaped clamp. The arms are connected at their proximal ends at apex 18. The distal ends of the arms form tips 24 and 26 which are spaced apart from each other in substantially parallel relationship for receiving the blood vessel.

As best shown in Figures 5 and 6, each arm is strengthened by upper and lower ribs 15 and 17. In addition, the arms are formed with a series of windows along their axial direction. A plurality of small windows 20 are spaced in the middle portion of each arm, and a single large window 22 is spaced toward the distal tip of each arm. The windows provide a series of ridges and openings for improved gripping of the blood vessel. The large windows also provide means to lock the track in a closed position as will be described below.

The apex of the track is shaped, as shown in Figures 4 and 7, to have a protruding rib 28, a bulbous portion 30 and a neck 32. A connecter 34 is secured on the apex, for connecting the track to the clip. As shown in Figure 5, reinforcing ribs 35 assist in securing the connector to the apex. The connector is shown in Figures 5 and 6 to have two posts 36 and 38 extending in opposite directions. With reference to Figure 4, the posts are shown as being "T"-shaped in cross-section, and having chamfered edges 40 and protrusions 41.

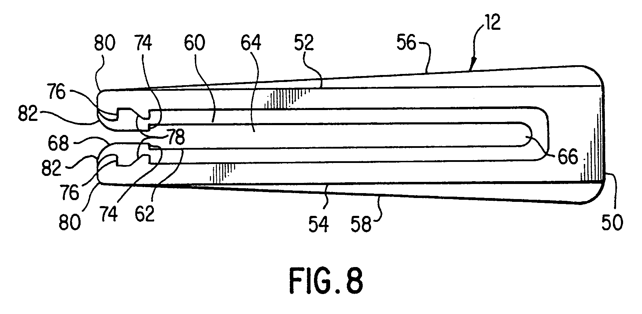

The clip 12 will be described in detail with reference to Figures 1 and 8. It is comprised generally of a U-shaped body 50 formed of cantilevered limbs 52 and 54. Tapered ribs 56 and 58 on an outside surface of the limbs provide strength, and interior ribs 60 and 62 define an extended slot 64 which extends from a crotch section 66 of the body at its proximal end to opening 68 at its distal end.

Distal ends of interior ribs 60 and 62 are shaped to form a key 74, or first contour surface. Each cantilevered limb 52 and 54 has a second contour surface formed of a notch 76 and chamfered surface 78, for engaging with complementary surfaces on the track. Distal tips 80 of the cantilevered limbs are shaped to have radial camming surfaces 82 for engaging the track in a manner described below.

When track 10 is connected to clip 12 as shown in Figure 1, the track is locked in the open position by virtue of the engagement between posts 36 and 38 of the track and the cantilevered limbs of the clip. More particularly, protruding portions 41 of the posts fit into notches 76 in the cantilevered limbs. In addition, chamfered edges 40 of the posts face the complementary chamfered surfaces 78 of the cantilevered limbs. At this position the track cannot be easily removed from the clip unless, for example, the cantilevered limbs are urged outwardly a substantial distance so the notches clear the protruding portions of the track. Thus, the chances of accidental disconnection of the track from the clip are relatively small.

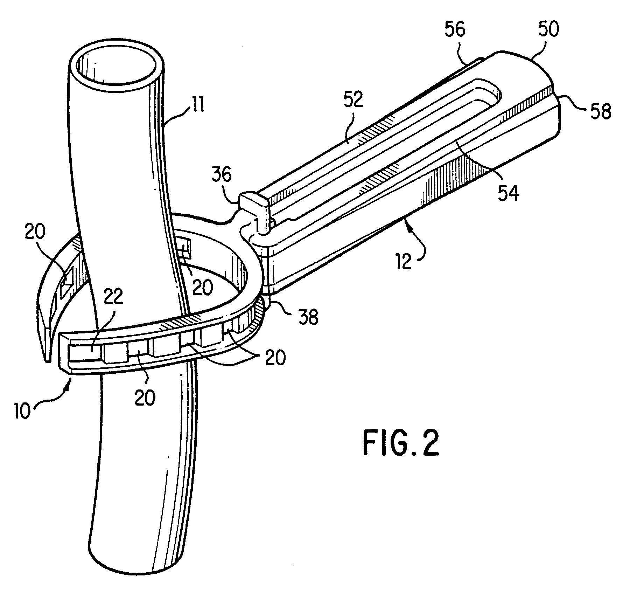

To close the ligation clip around the blood vessel, the track is held stationary by gripping posts 36 and 38 and the clip is slid forwardly toward the track. This sliding action urges the cantilevered limbs slightly outwardly and releases protruding portions 41 from notches 76 as the complementary chamfered edges 40 and chamfered surfaces 78 slide relative to each other. The sliding action also urges camming surfaces 82 of the cantilevered limbs against the curved arms 14 and 16 and begins to close the C-shaped clamp as shown in Figure 2.

The ligation clip is shown in the closed position in Figure 3. As will be appreciated, the closed position is achieved when the track completely occupies the elongated slot 64, with the posts 36 and 38 positioned at the proximal end of the clip. The ligation clip is locked in the closed position by engagement of keys 74 of the inner ribs in the large windows in the arms. Since the keys are shaped to be larger than the small windows, the ligation clip will only lock when the keys are registered with the large windows. Thus in the locked position of the clip, the blood vessel is securely ligated.

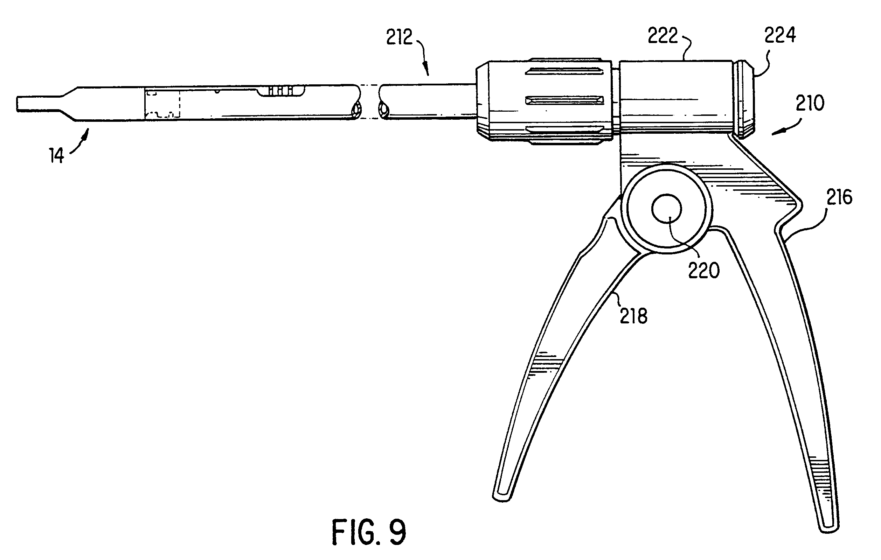

The ligation clip applicator of the present invention is shown in Figure 9 to comprise a handle assembly 210, an elongated and round or cylindrical neck portion 212 projecting from the handle assembly, and a ligation clip module 214 that can be mounted on the end of each neck portion. Generally speaking, the ligation clip is loaded into module 214 in a manner that will be described in more detail below and handle assembly 210 is actuated to close, or compress, a first part of the ligation clip around a vessel to be ligated.

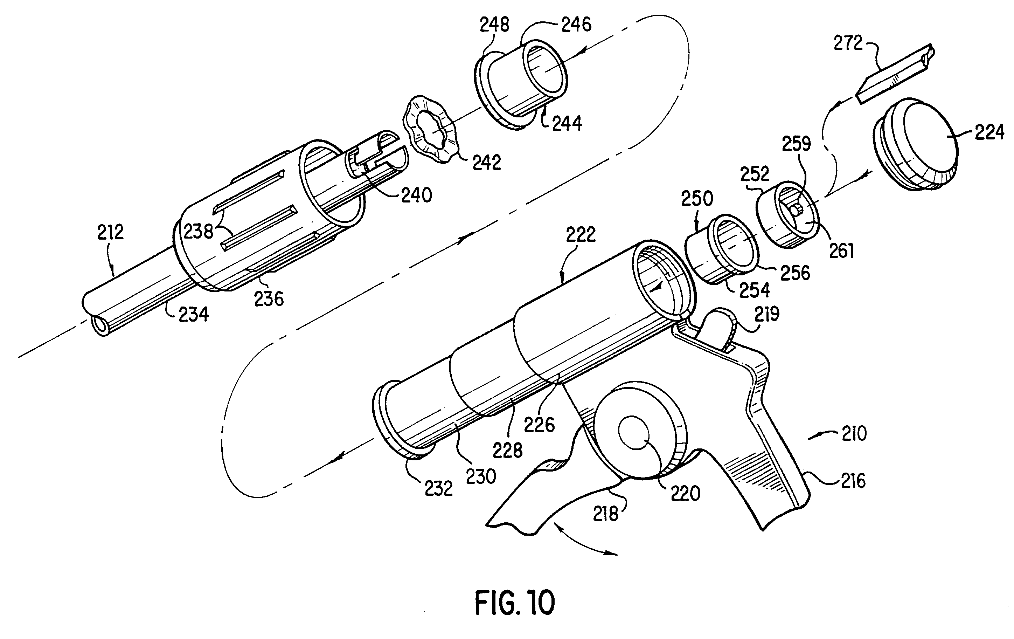

The handle assembly as shown in Figure 9 includes a handle 216 and a squeezable trigger 218 pivotally attached to the handle at pivot shaft 220. A barrel assembly 222 receives the proximal end of the neck portion 212. A threaded end cap 224 closes the proximal end of the barrel. As shown in Figure 10, the barrel assembly 222 is formed of a first barrel 226, a second barrel 228 and a third barrel 230 of progressively smaller diameters, that together form a telescope-like appearing structure. The smallest diameter barrel, third barrel 230, includes a radially projecting lip 232 at its distal end. The top or actuating end 219 of trigger 218 can also be seen in Figure 10.

The proximal end of the elongated neck portion shown in Figure 10 includes a hollow tube or shaft 234 and a collar 236 secured thereto and open at its proximal end. The shaft and collar, as well as the entire handle assembly, are preferably made of high grade surgical steel, such as, for example, stainless steel. The collar includes longitudinal ridges 238 so that it can be gripped easily to rotate the hollow shaft 234 about its longitudinal axis. The proximal end of the hollow shaft has two oppositely disposed J-shaped notches 240, commonly known as "bayonet locks", to secure the shaft in the handle assembly for such rotation in the manner described below.

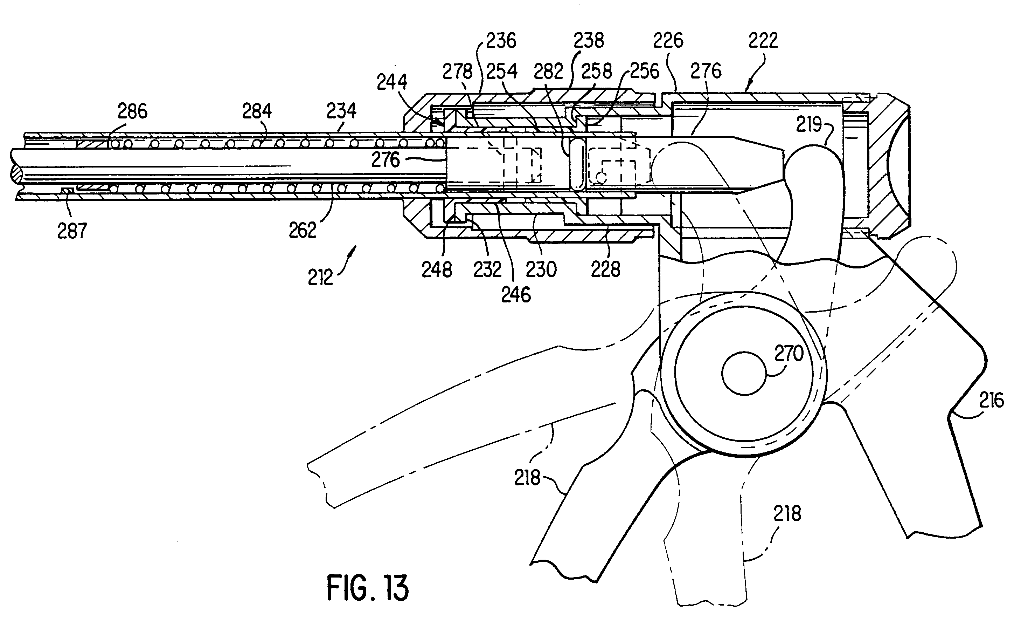

To assemble the neck portion and handle assembly, a resilient wave washer 242 and a first bushing 244 are placed over the proximal end of hollow shaft 234. The first bushing has a cylindrical portion 246 and a radially projecting rim 248 at its distal end. The hollow shaft is inserted into the open distal end of the barrel assembly 222 until the rim 248 on the first bushing 244 abuts the circumferential lip 232 on third barrel 230 as can be seen in detail in Figure 13. A second bushing 250 having a cylindrical portion 254 and a circumferential rim 256, and bayonet ring lock 252 are inserted into the open proximal end of the barrel assembly and over the end of the hollow shaft.

Referring again to Figure 13, the second bushing is inserted until rim 256 abuts a radially projecting ridge 258 formed between the second and third barrels. As best seen in Figure 10, the bayonet ring lock includes two pins 259 protruding inwardly from its inner cylindrical surface 261. The pins are designed to fit into the J-shaped notches 240 and lock the elongated neck portion in the handle assembly as shown in Figure 13.

In assembly the hollow shaft 234 and bayonet ring lock 252 are manipulated to guide the pins along the straight section of the J-shaped grooves. When the pins reach the end of the straight portion the hollow shaft is rotated relative to the bayonet ring lock and then moved in a reverse axial direction thereto to position the pins in the tip, or toe, section of the J-shaped grooves and lock the hollow shaft in the handle assembly. The resilient wave washer 242 is compressed between the inner radially projecting proximal surface of the collar 236 and the distal surface of the rim 248 to urge the neck portion assembly forwardly. As will be appreciated, with this assembly the neck portion is freely rotatable about its longitudinal axis while being secured to the handle assembly.

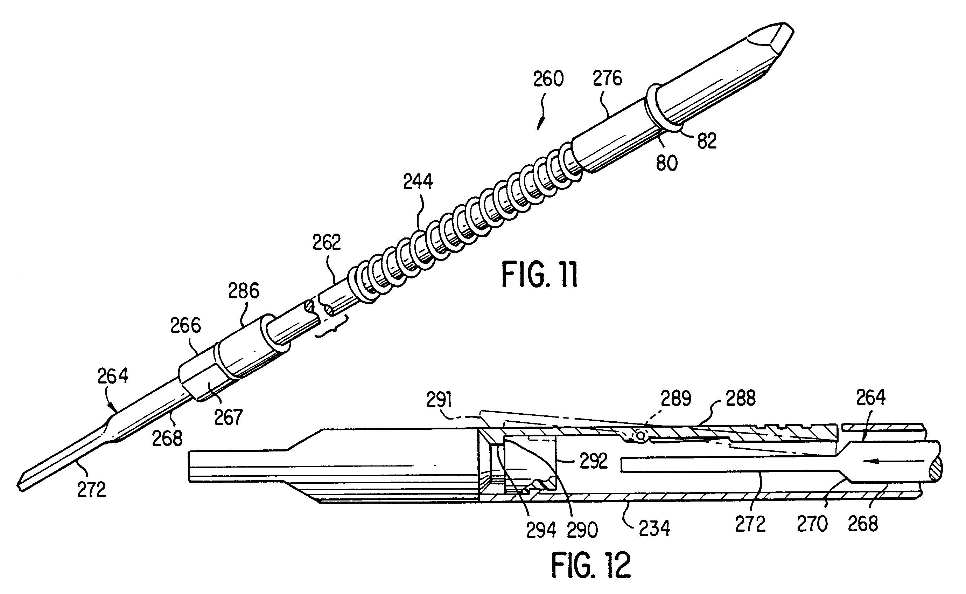

Figure 11 shows a sliding advancing mechanism, or advancer, 261 housed within hollow shaft 234 of the neck portion. The advancer is comprised of a cylindrical advancing shaft 262 connected at its distal end to an actuator 264. As shown in Figures 11 and 12, the actuator has a cylindrical section 268, on which a bearing 266 can ride and that tapers to a flat blade-like portion 272 having a rectangular cross-section. The bearing 266 is generally cylindrical but includes at least one flat surface 267 on its outer periphery running its entire axial length. The proximal end of advancing shaft 262 is secured to a cylindrical shaft adapter 276 by a transverse pin 278 as shown in Figure 13. The diameter of the shaft adapter approximates the inner diameter of hollow shaft 234. The shaft adapter also has a circumferential notch 280 in which resilient O-ring 282 is mounted, for stabilizing axial movement of the advancer within the hollow shaft and to act as a gaseous seal.

The advancer is urged toward the proximal end of the neck portion by a compression return spring 284 and sliding bushing 286. Both the spring and the barrel slide freely on the advancing shaft 262, with the proximal end of the spring abutting shaft adapter 276.

The advancer is assembled in the neck by inserting it in the proximal end of the barrel assembly. As shown in Figure 13, a short stop pin 287 extends inwardly from the inner surface of hollow shaft 234 in an intermediate region. When the advancer is inserted through the hollow shaft, the flat surface 267 of bearing 266 must oppose the stop pin 287 to permit it to slide past the pin and enable the advancer to be completely inserted into the hollow shaft. However, the sliding bushing 286, by virtue of its being cylindrical, cannot slide past the stop pin, and thus becomes arrested at that position. Further insertion of the advancer causes the spring 284 to engage the shaft adapter 276, thereby biasing the advancer in the rearward direction against the actuating end 219 of the trigger. When the advancer is fully inserted into the neck portion, the end cap 224 is placed on the barrel assembly.

Of course, other means for securing the advancer in the hollow shaft 234 and biasing it in the rearward direction are within the scope of the invention.

As can be seen in Figure 13, when the trigger 218 is pivoted in the counterclockwise direction about the pivot shaft 220 the top or actuating end 219 of the trigger engages the proximal end of the shaft adapter 276 thereby to urge the advancer assembly forwardly against the biasing force of the spring 284.

The shaft adapter 276, bushing 286 and bearing 266 of the advancer are preferably made of a non-metallic material, such as, for example, a hard plastic or a similar type polymer. In this way, sliding contact between the advancer and the hollow shaft is between non-metallic and metallic parts, thus reducing wear and providing smooth operation of the applicator. Alternatively, a similar result can be obtained using parts coated with a friction reducing material such as titanium nitride.

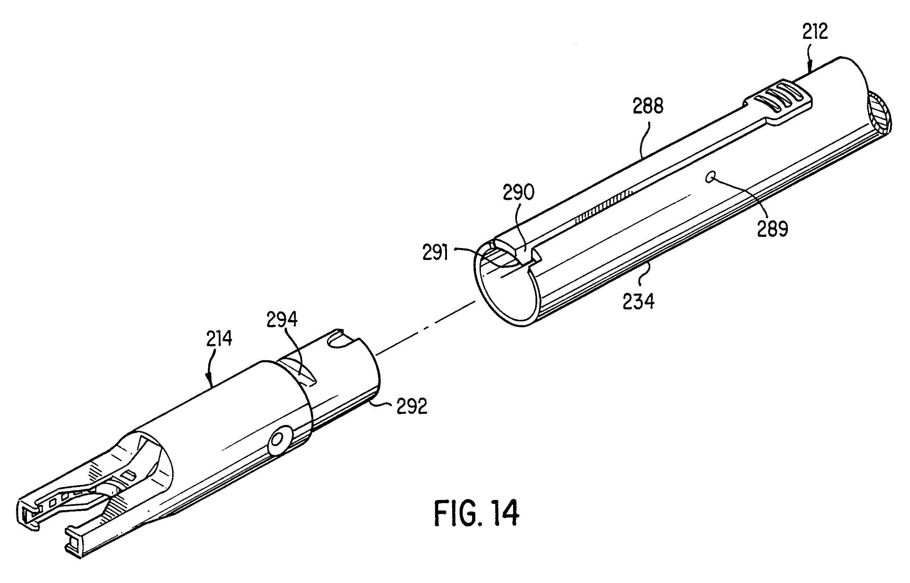

Figures 12 and 14 illustrate how the ligation clip module 214 is attached to the distal end of hollow shaft 234. Referring specifically to Figure 14, a spring-loaded latch 288 is pivotably mounted on a pivot pin 289 carried in wall of the shaft 234. The distal end of the latch is provided with a pawl 290. The module has a reduced diameter section 292 which fits into the open end of the hollow shaft. To secure the module in the hollow shaft, the proximal end of the latch is pressed down to raise the pawl as shown in phantom lines in Figure 12. The module is then inserted and the latch is released so the pawl catches a transverse groove 294 in the reduced diameter section to securely lock the module in the neck portion. Advantageously, the pawl 290 of the latch can be provided with a cam surface 291 that rides up on the proximal extreme of the reduced diameter section 292 of the module. In this way, the latch surface 292 of the pawl is automatically guided to the groove 294 when the module is inserted into the neck portion.

As a safety feature, when the latch is pivoted in the open position as shown in Figure 12, the proximal end of the latch abuts a tapered portion 270 of the actuator and prevents the advancer from moving farther forwardly in the hollow shaft.

Similarly, when the module is properly mounted in the neck portion and the actuator 264 is advanced to apply a ligation clip, as described below, the cylindrical section 268 of the actuator prevents the proximal end of the latch from being depressed to release the module.

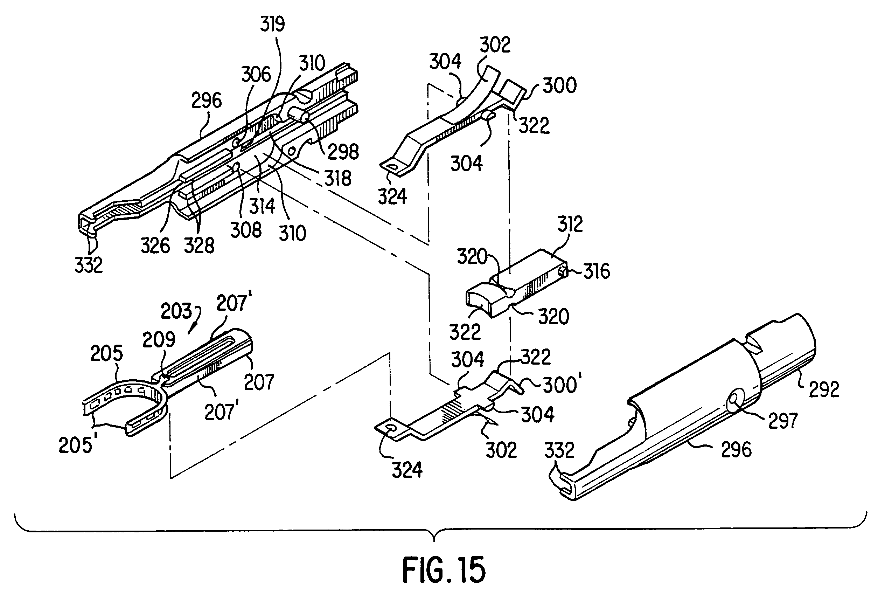

The individual components of the ligation clip module 214 are shown in Figure 15. The module housing includes two substantially identical cartridge halves 296. The cartridge halves are joined together by inserting an alignment pin 298 in one cartridge half into an unseen alignment hole in the other cartridge half and preferably gluing or otherwise securing the halves together. Upper and lower latches 300 and 300' each have an integral spring 302, and opposing trunions 304 which pivot in holes 306 and 308 in both cartridge halves. The integral springs press against ribs 310 in each cartridge half and urge the proximal ends of the latches toward each other.

A pusher 312 rides in a track 314 provided in the opposing cartridge halves. Laterally extending bosses 316 also align with axial grooves 318 within each cartridge half and act as stops to limit forward movement of the pusher by engaging the distal extremes 319 of the grooves 318. Nevertheless, the bosses 316 are designed to shear off to permit the clip to be forced out of the module after it is closed if the clip does not otherwise release from the module. The pusher includes a pair of transverse slots 320, one on each of its sides, for receiving a V-shaped bend 322 in each latch 300, 300'. In the rest or unfired position of the cartridge, the proximal ends of the latches 300, 300' are spaced apart by the pusher with the V-shaped bends received in the slots 320, thereby to hold the distal ends of the latches relatively close together. In addition, the front of the pusher has a curved portion 322 with a radius to match the rear end of the ligation clip as will be described below.

A window 297 in each cartridge half allows the user to view the pusher when it is in the pre-actuated, or ready-to-fire, position.

While the batches are preferably made of metal, the other parts of the module can be formed of plastic or other similar material. As will also be appreciated, the module is disposable. In addition, different size modules for mounting various size ligation clips can be easily connected to the same hollow shaft by using a standard size reduced diameter section.

For ease of description, the ligation clip will be described again, this time in cooperation with the applicator.

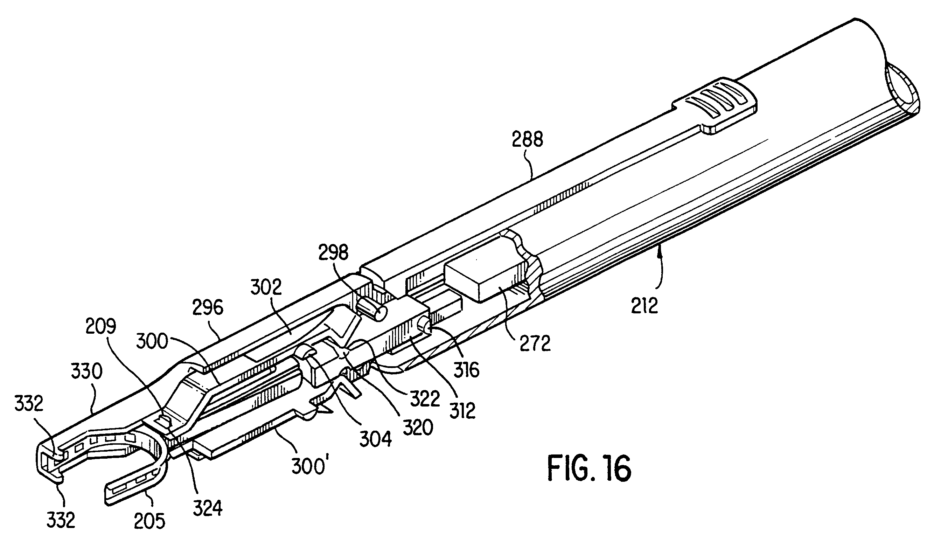

Figure 16 shows the ligation clip loaded in the ligation clip applicator and ready to be fired. In this attitude, the clip body 207 is positioned within the raceway and the Y-shaped clamp is protected within opposing flanges 330 and extended tips or hooks 332 of the cartridge halves 296 only one of each of which is shown in Figure 16. The opposed hooks of the assembled cartridge halves can be used to manipulate tissue when positioning a clip about a vessel to be ligated. The clamp is also held stationary by the posts 209 which are engaged in holes 324 in the latches. The pusher 312 is positioned behind the clip body and will slide forwardly upon actuation by the flat portion 272 of the advancer.

With the applicator loaded with a module holding the ligation clip and ready to be fired, the ligation clip may be positioned around the vessel to be ligated. Because of the slender and elongated design of the neck portion, the ligation clip applicator is ideally suited for use in minimally invasive surgical techniques. For example, the applicator can be used with such techniques by inserting the ligation clip carried in the clip module and the attached neck portion through a cannula in the patient to where the vessel to be ligated is located. The O-ring 282, which seals between the shaft 234 and the advancer assembly, acts as an internal gaseous sealing means for the applicator in such procedures.

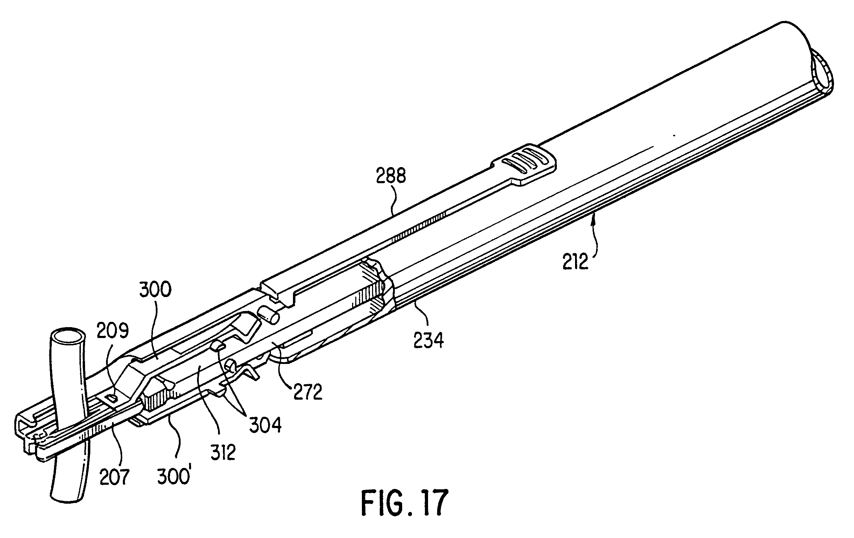

In Figure 17, the ligation clip is shown in position around the vessel to be ligated after the trigger 218 has been squeezed to slide the advancer, and through their mutual abutment, the pusher forwardly in a firing stroke. The flat portion 272 of the advancer forces the pusher in the forward direction, which in turn advances the clip body 207 forwardly to engage and compress the Y-shaped clamp, which is held in a fixed position by engagement of the latches, around the vessel. In this position, the bosses 316 of the pusher are visible in windows 297 of the cartridge housing, confirming that the ligation clip has been fired. As can be seen in Figure 17, the flat portion 272 of the advancer assumes the position of the pusher between the V-shaped bends in the latches 300, 300' to hold their distal ends together, to continue to secure the Y-shaped clamp of the ligation clip in its fixed position.

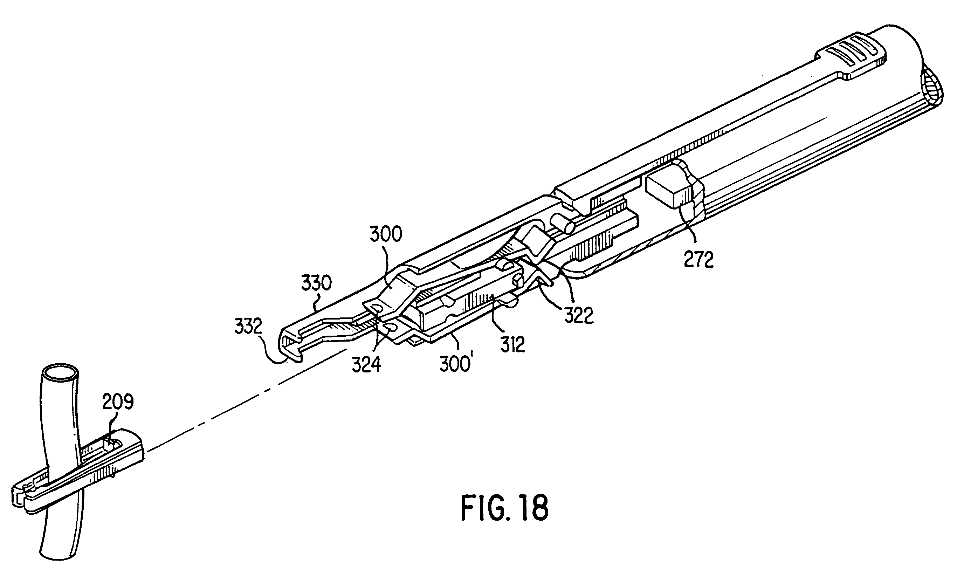

When the trigger is released, the advancer retreats in a return stroke in the rearward direction due to the biasing force of compression spring 284. However, as shown in Figure 18, this action leaves the pusher in the forward position in the cartridge, and therefore no obstruction is provided opposite to the V-shaped bends 322 in the latches. As a result, the biasing force of the integral springs collapses the latches together at their proximal ends. The distal ends of the latches thus separate and release engagement with the posts 209 of the Y-shaped clamp. The ligation clip can then freely slide out of the module. The fired module can thereafter be released from the hollow shaft by pivoting the latch 288 and removing it from the hollow neck shaft 212 to be discarded. A new module can then be inserted and secured in the hollow shaft and the applicator is then ready to be used again.

Accordingly, the present invention provides a unique and novel two-part ligation clip and a device for reliably and efficiently applying such two-part ligation clip to vessels to be ligated.

Although specific embodiments of the present invention have been described above in detail, it will be understood that this description is merely for purposes of illustration. Various modifications of and equivalent structures corresponding to the disclosed aspects of the preferred embodiments in addition to those described above may be made by those skilled in the art without departing from the present invention which is defined in the following claims, the scope of which is to be accorded the broadest interpretation so as to encompass such modifications and equivalent structures.

## Claims

1. A surgical ligation clip, including a track (10) having two arms (14,16) for engaging around a vessel to be ligated, said arms being joined together at a proximal end (18) of said track; a clip (12) having an extended slot (64) for slidably receiving said track; and a first locking means (41,76) for locking said track in an open position in said clip, characterized by a second locking means (74) for locking said track in a closed position in said clip.

2. A surgical ligation clip according to Claim 1, wherein said second locking means (74) includes a first complementary surface at a distal end of each said arm (24,26) and a first contour surface at a distal end (80) of said clip (12).

3. A surgical ligation clip according to Claim 1, wherein said first locking means (41,76) includes a connector (34) connected to the proximal end of said track (10) and having a second complementary surface and a second contour surface at the distal end (80) of said clip (12).

4. A surgical ligation clip according to Claim 3, wherein said first complementary surface is formed by large windows disposed at distal ends of said arms (24,26), and further comprising a pair of keys (74) forming said first contour surface at the distal end (80) of said clip (12).

5. A surgical ligation clip according to Claim 4, wherein said connector comprises two posts (36,38) each having a substantially T-shaped cross-section and chamfered edges (40) to form said second complementary surface, and said second contour surfaces are formed by a notched portion (76) contiguous with a chamfered surface (78) at the distal end (80) of said clip (12).

6. A surgical ligation clip according to Claim 1, wherein said arms (14,16) are curved and together form a substantially C-shaped track in the open position.

7. A surgical ligation clip according to Claim 1, wherein said clip (12) is formed by two cantilevered limbs connected at a proximal end to form a substantially U-shaped clip (12).

8. The surgical ligation clip of Claim 1 in combination with a ligation clip applicator for applying the two-part ligation clip of Claim 1, to a vessel (11); said applicator comprising:
a module (214) attached to the distal end of a hollow shaft (234) of the applicator for supporting said two-part ligation clip, said module including holding means for holding a first part of said ligation clip in a stationary position relative to said applicator, and sliding means for engaging and advancing a second part of said ligation clip relative to said first part to close said first part; and
actuating means (210,212), mountable with said module, for actuating said sliding means.

9. The combination according to Claim 8, wherein said holding means comprises a pair of latches housed in said module for gripping said first part of the ligation clip.

10. The combination according to Claim 9, wherein said first part of said ligation clip has at least one transversely projecting post and wherein at least one of said latches is formed with a hole for receiving said post thereby to provide said gripping.

11. The combination according to Claim 8, wherein said sliding means comprises a pusher slidable axially to advance within said module upon actuation by said actuating means.

12. The combination according to Claim 8, wherein said actuating means comprises a handle assembly including a pivotally mounted trigger (218) and a neck portion, said neck portion including a hollow shaft (234) and a sliding advancer (261) slidably mounted in said shaft, said trigger (218) being associated with said advancer (261) to drive said advancer in a forward direction to actuate said sliding means when said trigger (218) is pivoted.

13. The combination according to Claim 12, further comprising advancer biasing means (284) for biasing said advancer (261) in a rearward direction.

14. The combination according to Claim 12, further comprising a connecting latch pivotally mounted in the region of the distal end of said hollow shaft (234), said latch being formed to engage said module in a closed position, and in an open position to abut said advancer and prevent it from sliding in the forward direction.

15. The combination according to Claim 8, wherein said module is formed with opposing flange means for embracing at least a portion of the first part of said ligation clip, said flange means terminating in opposing inwardly directed hook means for manipulating tissue to be ligated.

## Patentansprüche

1. Chirurgische Abbindeklammer mit einer Bahn (10) mit zwei Armen (14, 16) für Angriff um ein abzubindendes Gefäß herum, wobei diese Arme am proximalen Ende (18) der Bahn zusammengefügt sind; einer Klammer (12) mit einem langgestreckten Schlitz (64) zum verschiebbaren Aufnehmen der Bahn; und einer ersten Verriegelungseinrichtung (41, 76) zum Verriegeln der Bahn in einer offenen Stellung der Klammer, **gekennzeichnet durch** eine zweite Verriegelungseinrichtung (74) zum Verriegeln der Bahn in einer geschlossenen Stellung der Klammer.

2. Chirurgische Abbindeklammer nach Anspruch 1, bei der die zweite Verriegelungseinrichtung (74) eine erste komplementäre Fläche am distalen Ende jedes Arms (24, 26) und eine erste Konturfläche am distalen Ende (80) der Klammer (12) aufweist.

3. Chirurgische Abbindeklammer nach Anspruch 1, bei der die erste Verriegelungseinrichtung (41, 76) einen Verbinder (34) aufweist, der mit dem proximalen Ende der Bahn (10) verbunden ist und eine zweite komplementäre Fläche und eine zweite Konturfläche aufweist, mit einer zweiten Konturfläche am distalen Ende (80) der Klammer (12).

4. Chirurgische Abbindeklammer nach Anspruch 3, bei der die erste komplementäre Fläche aus großen Fenstern besteht, die an distalen Enden der Arme (24, 26) angeordnet sind, ferner mit einem Paar Verankerungen (74), die die erste Konturfläche am distalen Ende (80) der Klammer (12) bilden.

5. Chirurgische Abbindeklammer nach Anspruch 4, bei der der Verbinder zwei Stifte (36, 38) aufweist, von denen jeder im wesentlichen T-förmigen Querschnitt und abgeschrägte Kanten (40) aufweist, um die zweite komplementäre Fläche zu bilden, und die zweiten Konturflächen durch einen eingekerbten Abschnitt (76) gebildet sind, der mit einer abgeschrägten Fläche (78) am distalen Ende (80) der Klammer (12) zusammenhängt.

6. Chirurgische Abbindeklammer nach Anspruch 1, bei der die Arme (14, 16) gekrümmt sind und zusammen in der offenen Stellung eine im wesentlichen C-förmige Bahn bilden.

7. Chirurgische Abbindeklammer nach Anspruch 1, bei der die Klammer (12) aus zwei freitragenden Schenkeln besteht, die am proximalen Ende verbunden sind, um eine im wesentlichen U-förmige Klammer (12) zu bilden.

8. Chirurgische Abbindeklammer nach Anspruch 1 in Kombination mit einem Applikator für Abbindeklammern zum Anlegen der zweiteiligen Abbindeklammer von Anspruch 1 an ein Gefäß (11), wobei der Applikator folgendes aufweist:
- ein Modul (214), das am distalen Ende eines hohlen Schafts (234) des Applikators befestigt ist, um die zweiteilige Abbindeklammer aufzunehmen, und das eine Halteeinrichtung zum Halten eines ersten Teils der Abbindeklammer in einer stationären Stellung relativ zum Applikator sowie eine Verschiebeeinrichtung zum Angreifen an einem zweiten Teil der Abbindeklammer und zum Vorschieben derselben relativ zum ersten Teil, um diesen ersten Teil zu schließen, aufweist; und
- eine Betätigungseinrichtung (210, 212), die am Modul montierbar ist, um die Verschiebeeinrichtung zu betätigen.

9. Kombination nach Anspruch 8, bei der die Halteeinrichtung ein Paar Klinken aufweist, die im Modul untergebracht sind, um am ersten Teil der Abbindeklammer anzugreifen.

10. Kombination nach Anspruch 9, bei der der erste Teil der Abbindeklammer mindestens einen quer vorspringenden Stift aufweist, wobei mindestens eine der Klinken mit einem Loch zum Aufnehmen des Stifts versehen ist, um dadurch für dieses Angreifen zu sorgen.

11. Kombination nach Anspruch 8, bei der die Verschiebeeinrichtung einen Vordrücker aufweist, der axial verschiebbar ist, um bei Betätigung der Betätigungseinrichtung innerhalb des Moduls nach vorne zu laufen.

12. Kombination nach Anspruch 8, bei der die Betätigungseinrichtung eine Handgriffanordnung mit einem verschwenkbar montierten auslöser (218) und einem Halsabschnitt aufweist, der einen hohlen Schaft (234) und eine innerhalb dieses Schafts verschiebbar angebrachte Verschiebeinrichtung (261) aufweist, wobei der Auslöser (218) der Verschiebeinrichtung (261) so zugeordnet ist, dass er diese in der Richtung nach vorne verschiebt, um die Gleiteinrichtung zu betätigen, wenn der Auslöser (218) verschwenkt wird.

13. Kombination nach Anspruch 12, ferner mit einer Vorbelastungseinrichtung (284) für die Vorschubeinrichtung zum Vorbelasten dieser Vorschubeinrichtung (261) in Rückwärtsrichtung.

14. Kombination nach Anspruch 12, ferner mit einer Verbindungsklinke, die schwenkbar im Bereich des distalen Endes des hohlen Schafts (234) angebracht ist und so ausgebildet ist, dass sie am Modul in geschlossener Stellung angreift, während sie in offener Stellung an der Vorschubeinrichtung anschlägt und diese an einem Verschieben in Vorwärtsrichtung hindert.

15. Kombination nach Anspruch 8, bei der das Modul mit gegenüberstehenden Flanscheinrichtungen versehen ist, um zumindest einen Abschnitt des ersten Teils der Abbindeklammer zu umfassen, wobei diese Flanscheinrichtungen in einander gegenüberstehenden, nach innen gerichteten Hakeneinrichtungen enden, um abzubindendes Gewebe zu handhaben.

## Revendications

1. Pince de ligature chirurgicale comportant une agrafe (10) ayant deux bras (14, 16) destinés à venir en contact autour d'un vaisseau à ligaturer, lesdits bras étant reliés ensemble au niveau d'une extrémité proximale (18) de ladite agrafe ; une pince (12) ayant une fente étendue (64) pour recevoir de manière coulissante ladite agrafe ; et des premiers moyens de verrouillage (41, 76) pour verrouiller ladite agrafe dans une position ouverte dans ladite pince, caractérisée par des seconds moyens de verrouillage (74) pour verrouiller ladite agrafe dans une position fermée de ladite pince.

2. Pince de ligature chirurgicale selon la revendication 1, dans laquelle lesdits seconds moyens de verrouillage (74) comportent une première surface complémentaire au niveau d'une extrémité distale de chaque dit bras (24, 26) et une première surface de profil au niveau d'une extrémité distale (80) de ladite pince (12).

3. Pince de ligature chirurgicale selon la revendication 1, dans laquelle lesdits premiers moyens de verrouillage (41, 76) comportent un connecteur (34) relié à l'extrémité proximale de ladite agrafe (10) et ayant une seconde surface complémentaire et une seconde surface de profil au niveau de l'extrémité distale (80) de ladite pince (12).

4. Pince de ligature chirurgicale selon la revendication 3, dans laquelle ladite première surface complémentaire est constituée de grandes fenêtres agencées au niveau des extrémités distales desdits bras (24, 26), et comporte de plus deux clavettes (74) formant ladite première surface de profil au niveau de l'extrémité distale (80) de ladite pince (12).

5. Pince de ligature chirurgicale selon la revendication 4, dans laquelle ledit connecteur comporte deux montants (36, 38) ayant chacun une section transversale à peu prés en forme de T et des bords chanfreinés (40) pour former ladite seconde surface complémentaire, et lesdites secondes surfaces de profil sont formées par une partie munie d'une encoche (76) contigué à une surface chanfreinée (78) au niveau de l'extrémité distale (80) de ladite pince (12).

6. Pince de ligature chirurgicale selon la revendication 1, dans laquelle lesdits bras (14, 16) sont incurvés et forment ensemble une agrafe à peu près en forme de C dans la position ouverte.

7. Pince de ligature chirurgicale selon la revendication 1, dans laquelle ladite pince (12) est munie de deux branches en porte à faux reliées au niveau d'une extrémité proximale pour former une pince à peu près en forme de U (12).

8. Pince de ligature chirurgicale selon la revendication 1, en combinaison avec un applicateur de pince de ligature pour appliquer la pince de ligature en deux parties de la revendication 1 sur un vaisseau (11) ; ledit applicateur comportant :
un module (214) fixé sur l'extrémité distale d'un arbre creux (234) de l'applicateur pour supporter ladite pince de ligature en deux parties, ledit module comportant des moyens de maintien pour maintenir une première partie de ladite pince de ligature dans une position stationnaire par rapport audit applicateur, et des moyens de coulissement pour venir en contact avec une seconde partie de ladite pince de ligature et la faire avancer par rapport à ladite première partie pour fermer ladite première partie ; et
des moyens d'actionnement (210, 212), pouvant être munis dudit module, pour actionner lesdits moyens de coulissement.

9. Combinaison selon la revendication 8, dans laquelle lesdits moyens de maintien comportent une paire de verrous reçue dans ledit module pour saisir ladite première partie de la pince de ligature.

10. Combinaison selon la revendication 9, dans laquelle ladite première partie de ladite pince de ligature a au moins un montant faisant saillie transversalement et dans laquelle au moins un desdits verrous est muni d'un trou destiné à recevoir ledit montant en assurant ainsi ladite saisie.

11. Combinaison selon la revendication 8, dans laquelle lesdits moyens de coulissement comportent un poussoir pouvant coulisser axialement pour avancer dans ledit module lors de l'actionnement par lesdits moyens d'actionnement.

12. Combinaison selon la revendication 8, dans laquelle lesdits moyens d'actionnement comportent un ensemble de poignée comportant une gâchette agencée de manière pivotante (218) et une partie formant col, ladite partie formant col comportant un arbre creux (234) et un dispositif d'avancement coulissant (261) agencé de manière coulissante dans ledit arbre, ladite gâchette (218) étant associée audit dispositif d'avancement (261) pour entraîner ledit dispositif d'avancement dans une direction vers l'avant pour actionner lesdits moyens de coulissement lorsque ladite gâchette (218) pivote.

13. Combinaison selon la revendication 12, comportant de plus des moyens de rappel du dispositif d'avancement (284) pour rappeler ledit dispositif d'avancement (261) dans une direction vers l'arrière.

14. Combinaison selon la revendication 12, comportant de plus un verrou de liaison agencé de manière pivotante dans la zone de l'extrémité distale dudit arbre creux (234), ledit verrou étant formé pour venir en prise avec ledit module dans une position fermée, et, dans une position ouverte, pour venir en butée contre ledit dispositif d'avancement et empêcher celui-ci de coulisser dans la direction vers l'avant.

15. Combinaison selon la revendication 8, dans laquelle ledit module est muni de moyens formant rebords opposes pour entourer au moins une partie de la première partie de ladite pince de ligature, lesdits moyens formant rebords se terminant en moyens formant crochets dirigés vers l'intérieur, opposés, pour manipuler les tissus à ligaturer.
